# EUROPEAN PATENT APPLICATION

(11) **EP 4 563 566 A1**
(43) Date of publication of application: **04.06.2025**
(21) Application number: 23856273.0
(22) Date of filing: 28.06.2023
(51) Int. Cl.: C07C 57/03, C07C 57/04, C07C 57/18, C07C 59/40, C07C 211/21, C07C 211/23, C07C 321/18, G03F 7/004, H01L 21/00

(54) **LIGAND COMPOUND, COMPLEX AND USE THEREOF**

(30) Priority: 22.08.2022 CN 202211009142
(71) Applicant: Huawei Technologies Co., Ltd., Shenzhen, Guangdong 518129 (CN); Najing Technology Corporation Limited, Hangzhou, Zhejiang 310052 (CN)
(72) Inventor: LI, Zhe, Shenzhen, Guangdong 518129 (CN); GAO, Yuan, Hangzhou, Zhejiang 310052 (CN); DING, Xianyu, Hangzhou, Zhejiang 310052 (CN)
(74) Representative: Körber, Martin Hans
(86) International application number: PCT/CN2023/103439
(87) International publication number: WO 2024/041182

(57) **Abstract**

This application provides a ligand compound. A structure of the ligand compound is X-Y-L-(Z)ₙ. X is HOOC-, NH₂-, HOOOP-, HSS-, or HS-; Y is at least one of a single bond, an aliphatic organic group, an aromatic organic group, and an organic group containing at least one of an ester group, an ether group, a carbonyl group, and an amide group; L is an atom or an organic group for forming at least three chemical bonds; Z is an organic group with an end-capping group being a crosslinkable group; and n is an integer greater than or equal to 2. This application further provides a complex containing the ligand compound and a solution thereof, a patterned light-emitting layer using the complex and a preparation method thereof, an apparatus using the patterned light-emitting layer, and an electronic device. The ligand compound has high photoactivity, which can reduce an exposure dose and shorten exposure duration, thereby improving patterning efficiency.

## Description

### CROSS-REFERENCE TO RELATED APPLICATIONS

This application claims priority to Chinese Patent Application No. 202211009142.5, filed with the China National Intellectual Property Administration on August 22, 2022 and entitled "LIGAND COMPOUND, COMPLEX, AND USE THEREOF", which is incorporated herein by reference in its entirety.

### TECHNICAL FIELD

This application relates to the field of photosensitive semiconductor technologies, and in particular, to a ligand compound, a complex containing the ligand compound and a complex solution, a patterned light-emitting layer using the complex and a preparation method thereof, a light-emitting apparatus using the patterned light-emitting layer, and an electronic device using the light-emitting apparatus.

### BACKGROUND

A semiconductor light-emitting layer (for example, a quantum dot light-emitting layer) in an electroluminescent (or photoluminescent) apparatus usually needs to be patterned. Methods for patterning the semiconductor light-emitting layer in the industry mainly include a transfer printing method and a photoresist etching method. However, the transfer printing method is limited in a processing size, and therefore cannot implement patterning of micro-sized products, resulting in low patterning fineness and a low processing yield. In addition, the photoresist etching method has a complex process, and a developing solution and an etching solution have an adverse impact on the semiconductor light-emitting layer and another functional layer (such as a hole transport layer or an electron transport layer), consequently affecting a yield of the electroluminescent (or photoluminescent) apparatus.

### SUMMARY

In view of this, to resolve at least one of the foregoing defects, it is necessary for embodiments of this application to provide a ligand compound with high photoactivity.

In addition, embodiments of this application further provide a complex containing the ligand compound and a solution thereof, a patterned light-emitting layer using the complex and a preparation method thereof, a light-emitting apparatus using the patterned light-emitting layer, and an electronic device using the light-emitting apparatus.

According to a first aspect of embodiments of this application, a ligand compound, where the ligand compound has a structure shown in the following formula (I):

X-Y-L-(Z)ₙ (I),

where
X is HOOC-, NH₂-, HOOOP-, HSS-, or HS-;
Y is at least one of a single bond, an aliphatic organic group, an aromatic organic group, and an organic group containing at least one of an ester group, an ether group, a carbonyl group, and an amide group;
L is an atom having a valence of at least three or an organic group having at least three chemical bonds;
Z is an organic group with an end-capping group being a crosslinkable group; and
n is an integer greater than or equal to 2.

In embodiments of this application, a plurality of organic groups with an end-capping group being a crosslinkable group are introduced to one end of a molecular main chain of the ligand compound, so that a quantity of crosslinkable groups on each ligand compound can be increased, thereby improving photoactivity of the ligand compound, which helps reduce an exposure dose and shorten exposure duration, thereby improving patterning efficiency.

In some embodiments, Z in formula (I) is selected from at least one of an alkenyl group, an alkynyl group, an allyl group, an acrylic group, an acryloyl group, an acrylamido group, an azido group, an azidophenyl group, a fluoro-substituted azidophenyl group, diazo carbon, benzophenone, and a cinnamyl group.

End-capping groups of the foregoing organic groups are double bonds or triple bonds. These end-capping groups have a crosslinkable function. A plurality of the foregoing organic groups are contained in formula (I), increasing a quantity of crosslinkable groups, which can further improve photoactivity of the ligand compound.

In some embodiments, L in formula (I) includes carbon or nitrogen.

Carbon is a tetravalent atom that can form four chemical bonds with another atom or organic group, and can be linked to two or three Zs with an end-capping group. Nitrogen is a trivalent atom that can form three chemical bonds with another atom or organic group, and can be linked to two Zs with an end-capping group. Therefore, when L in formula (I) includes carbon or nitrogen, two or three Zs may be linked to the ligand compound, which helps improve photoactivity of the ligand compound. In addition, in this case, the Z group has small steric hindrance for linkage, which helps introduce a plurality of Z groups in the ligand compound.

In some embodiments, Y in formula (I) is a saturated aliphatic organic group, and is selected from at least one of a saturated aliphatic hydrocarbyl group, aliphatic ester group, aliphatic amide group, aliphatic oxycarbonyl group, and aliphatic ether group.

By linking the saturated aliphatic organic group to the molecule of the ligand compound, flexibility of the molecular chain of the ligand compound can be improved, which helps contact between terminal crosslinkable groups of different ligand compounds to undergo a crosslinking reaction.

In some embodiments, the ligand compound has a structure shown in any one of chemical formulas (II) to (IV): 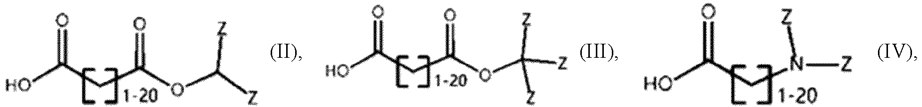 where Z in formulas (II) to (IV) is an organic group with an end-capping group being a crosslinkable group.

By linking two or three Zs with an end-capping group being a crosslinkable group to one end of a molecular main chain of the ligand compound, a quantity of active sites is increased, and further a chance of contact between crosslinkable groups on different semiconductor particles is increased, thereby improving photoactivity of the ligand compound, and crosslinking density can also be increased, thereby improving stability of a final crosslinked structure. In addition, when Y in formula (I) is a saturated aliphatic ester group with a number of carbon atoms of 1 to 22, in a synthesis process, a raw material of the Y structure has only a carboxyl group at an end, and a location of an active linking site is determined, which helps introduction of the organic group Z to the end of the main chain, making a synthesis path easier to implement, so that the ligand compound with the structure shown in formula (I) designed in this application is obtained.

In some embodiments, the crosslinkable group is a carbon-carbon double bond, and the ligand compound has a structure shown in any one of chemical formulas (V) to (XVI):

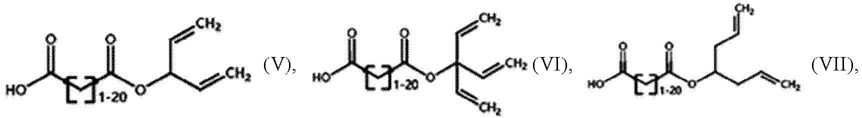

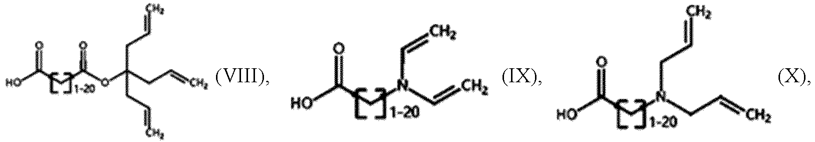

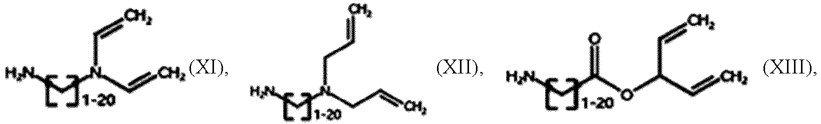

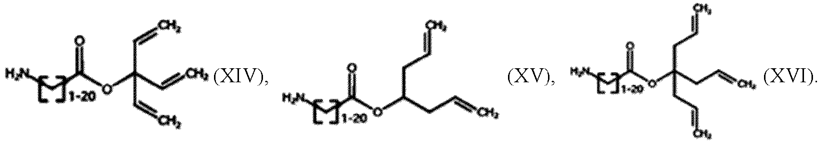

According to a second aspect of embodiments of this application, a complex is provided. The complex includes semiconductor particles and at least one of the foregoing ligand compound, where the ligand compound is coordinated to a surface of the semiconductor particles.

In embodiments of this application, a plurality of organic groups Z with crosslinking activity are linked to an end of a molecular main chain of the ligand compound, increasing a quantity of crosslinkable groups at the end of the molecular main chain of the ligand compound, which helps improve photoactivity. In addition, the crosslinkable group is located at an end of each organic group Z, increasing a chance of contact between crosslinkable groups on different complexes, which helps contact between crosslinkable groups of different semiconductor particle complexes to undergo crosslinking and curing reactions, further improving photoactivity, reducing an exposure dose, and shortening exposure duration, thereby improving patterning efficiency, and crosslinking density can be increased, improving stability of a finally formed crosslinked structure.

In some embodiments, the semiconductor particles include at least one of CdS, CdSe, CdTe, ZnS, ZnSe, ZnTe, PbS, PbSe, PbTe, HgS, HgSe, HgTe, GaN, GaP, GaAs, InP, InAs, ZnO, SnO₂, TiO₂, In₂O₃, Ga₂O₃, SiO₂, NiO, MoO₃, WO₃, Cu₂O, CuO, Fe₃O₄, Au, Ag, carbon dots, CsPbCl₃, CsPbBr₃, CsPbI₃, CH₃NH₃PbCl₃, CH₃NH₃PbBr₃, and CH₃NH₃PbI₃.

In some embodiments, an assistant dispersing ligand is further included, where the assistant dispersing ligand is coordinated to the surface of the semiconductor particles, and a weight of the ligand compound is 2% to 50% of a total weight of the assistant dispersing ligand and the ligand compound.

By adding the assistant dispersing ligand, dispersion of the new ligand compound synthesized in the foregoing embodiments can be improved, so that the new ligand compound can be uniformly linked to the surface of the semiconductor particles, further improving photoactivity of the semiconductor particles. In addition, by adding the assistant dispersing ligand, an amount of the new ligand compound can also be reduced while the photoactivity of the semiconductor particles is improved. Moreover, if an addition amount of the new ligand compound is excessively low (less than 2%), the improvement of the photoactivity of the semiconductor particles is limited; if an addition amount of the new ligand compound is excessively high (more than 50%), an amount of the assistant dispersing ligand is excessively low, which is not conducive to uniform dispersion of the new ligand compound, and the amount of the new ligand compound is increased, which increases costs of the new ligand compound. Therefore, in embodiments of this application, a weight proportion of the ligand compound is controlled within a range of 2% to 50%.

In some embodiments, the weight of the ligand compound is 5% to 30% of the total weight of the assistant dispersing ligand and the ligand compound.

In some embodiments, the assistant dispersing ligand includes at least one of fatty acid, unsaturated fatty acid, fatty amine, unsaturated fatty amine, alkylthiol, unsaturated alkylthiol, alkyl phosphonic acid, and unsaturated alkyl phosphonic acid.

The addition of the assistant dispersing ligand of the foregoing types to the complex helps reduce an amount of the new ligand compound and further reduce costs. In addition, after a small amount of the new ligand compound is formulated with the assistant dispersing ligand, the small amount of the new ligand compound can be uniformly attached to the surface of the semiconductor particles, and dispersion of the semiconductor particle complex in an organic solvent can be improved, facilitating film formation. Moreover, the formulation of the new ligand compound and the assistant dispersing ligand helps further improve photoactivity of the formulated ligand, reduce an exposure dose, and shorten exposure duration, thereby improving patterning efficiency.

According to a third aspect of embodiments of this application, a complex solution is further provided. The complex solution includes an organic solvent and the foregoing complex dispersed in the organic solvent.

According to a fourth aspect of embodiments of this application, a patterned light-emitting layer is provided. The patterned light-emitting layer includes a crosslinked structure, where the crosslinked structure is obtained through crosslinking of the foregoing complex.

Because a plurality of organic groups Z with crosslinking activity are linked to an end of a molecular main chain of the ligand compound, a quantity of crosslinkable groups on the surface of the complex is increased, thereby improving photoactivity of the ligand compound. In addition, the crosslinkable group is located at an end of each organic group Z, increasing a chance of contact between crosslinkable groups on different semiconductor particles, which helps contact between crosslinkable groups on different complexes to undergo crosslinking and curing, further improving photoactivity, reducing an exposure dose, and shortening exposure duration, thereby improving patterning efficiency. Moreover, due to the increase of a quantity of terminal crosslinkable groups, crosslinking density of a crosslinked network structure can be increased, improving stability of the crosslinked structure, and reducing damage to the crosslinked structure caused by a developing solution in a development process. Therefore, the obtained patterned light-emitting layer has high pattern fineness, and a uniform and adjustable thickness, and the patterned light-emitting layer can show good light-emitting features.

According to a fifth aspect of embodiments of this application, a method for preparing a patterned light-emitting layer is provided. The preparation method includes the following step:
patterning a film containing the foregoing complex, to obtain the patterned light-emitting layer, where the patterned light-emitting layer includes a crosslinked structure obtained through crosslinking of the complex.

Patterning is directly performed on the film containing the complex, so that a part of the complex that needs to be retained can undergo crosslinking and curing to form a crosslinked structure, and an unnecessary part of the complex does not need to undergo crosslinking, and thus the uncrosslinked part of the complex is easily removed. A formed pattern has high fineness, a process is simple with no need of a photoresist, and steps of the entire process are fewer than steps of a patterning process using a photoresist, with no need of a developing solution for the photoresist, which has little impact on the patterned light-emitting layer and a functional layer of a product, and is conducive to improving the quality and yield of the product. In addition, compared with a transfer printing method, the patterning process in embodiments of this application imposes a small limitation on a size of a product, which facilitates miniaturization of a formed product.

In some embodiments, the preparation method includes the following steps:
exposing the film containing the foregoing complex, to obtain an exposed film; and
developing the exposed film to obtain the patterned light-emitting layer.

The patterned light-emitting layer is formed through a direct photoetching process. A process is simple, and a fine pattern can be formed without a photoresist. In addition, steps of the entire process are fewer than steps of a patterning process using a photoresist, with no need of a developing solution for the photoresist, which has little impact on the patterned light-emitting layer and a functional layer of a product, and is conducive to improving the quality and yield of the product.

In some embodiments, time for exposing is 0.1 seconds to 600 seconds.

By forming the complex with the new ligand compound and the semiconductor particles, photoactivity of the complex can be effectively improved, thereby shortening exposure time and improving exposure efficiency.

In some embodiments, a light intensity for exposing is 0.001 mW/cm² to 1000 mW/cm².

By forming the complex with the new ligand compound and the semiconductor particles, photoactivity of the complex can be effectively improved, thereby reducing the light intensity for exposure.

According to a sixth aspect of embodiments of this application, a light-emitting apparatus is provided. The light-emitting apparatus includes the foregoing patterned light-emitting layer or the patterned light-emitting layer prepared by the foregoing preparation method.

The patterned light-emitting layer formed using the foregoing complex by the method for preparing the patterned light-emitting layer has high pattern fineness, and a uniform and adjustable thickness. A patterning process has little impact on a functional layer of the patterned light-emitting layer. The patterned light-emitting layer can show good light-emitting features, and may be used in various fields, including a semiconductor apparatus.

According to a seventh aspect of embodiments of this application, an electronic device is provided. The electronic device includes a housing and the foregoing light-emitting apparatus on the housing.

### BRIEF DESCRIPTION OF DRAWINGS

FIG. 1 is a diagram of a complex according to an embodiment of this application;
FIG. 2 is a diagram of a crosslinking process of a complex according to an embodiment of this application;
FIG. 3 is a diagram of a process of preparing a patterned light-emitting layer according to an embodiment of this application;
FIG. 4a is a diagram of a light-emitting apparatus according to an embodiment of this application;
FIG. 4b is a diagram of a light-emitting apparatus according to another embodiment of this application;
FIG. 5 is a diagram of an electronic device according to an embodiment of this application;
FIG. 6 is a hydrogen nuclear magnetic resonance spectrum of a ligand compound synthesized according to Synthesis Example 1 of this application;
FIG. 7 is a hydrogen nuclear magnetic resonance spectrum of a ligand compound synthesized according to Synthesis Example 2 of this application;
FIG. 8 is a hydrogen nuclear magnetic resonance spectrum of a ligand compound synthesized according to Synthesis Example 3 of this application;
FIG. 9 is a hydrogen nuclear magnetic resonance spectrum of a ligand compound synthesized according to Synthesis Example 4 of this application;
FIG. 10 is a graph of an ultraviolet-visible light absorption curve according to Embodiment 1 of this application;
FIG. 11 is a graph of an ultraviolet-visible light absorption curve according to Comparative Example 1 of this application;
FIG. 12 is a graph of an ultraviolet-visible light absorption curve according to Embodiment 2 of this application;
FIG. 13 is a graph of an ultraviolet-visible light absorption curve according to Comparative Example 2 of this application; and
FIG. 14 is a graph of an ultraviolet-visible light absorption curve according to Embodiment 5 of this application.

### DESCRIPTION OF REFERENCE NUMERALS OF MAIN ELEMENTS

| | |
|---|---|
| Complex film | 10 |
| Exposure region | 11 |
| Non-exposure region | 12 |
| Substrate | 001 |
| Anode layer | 20 |
| Photoetching mask | 30 |
| First functional layer | 40 |
| Second functional layer | 50 |
| Cathode layer | 60 |
| Patterned light-emitting layer | 100 |
| Upright component | 200 |
| Inverted component | 300 |
| Electronic device | 400 |
| Housing | 410 |

### DESCRIPTION OF EMBODIMENTS

The following describes embodiments of this application with reference to the accompanying drawings in embodiments of this application. Unless otherwise specified, a data range involved in this application should include an end value.

As the transfer printing method and photoresist etching method for patterning a semiconductor light-emitting material have the foregoing defects, the researchers further study a method for direct photoetching on the semiconductor light-emitting material. This method is coating a substrate with a semiconductor light-emitting material with a patterning function, exposing a part of the semiconductor light-emitting material to undergo crosslinking and curing, and then dissolving an uncrosslinked and uncured part of the semiconductor light-emitting material in a solvent, to form a patterned semiconductor light-emitting layer. The method for direct photoetching on the semiconductor light-emitting material has a simple process, provides high patterning fineness, can implement patterning of micro-sized products, and has no adverse impact on the semiconductor light-emitting layer and a functional layer (such as a hole transport layer or an electron transport layer), which helps increase a product yield.

However, since a semiconductor material has no photosensitivity, photoetching cannot be directly performed on the semiconductor light-emitting material, and the semiconductor material needs to be coordinated with a functional ligand having photosensitivity, to make the semiconductor material photosensitive. However, an existing functional ligand with photosensitivity has low photoactivity, and requires a large exposure dose and long exposure time.

Therefore, embodiments of this application provide a complex with high photoactivity that can reduce an exposure dose and shorten exposure duration to improve patterning efficiency. The complex is a semiconductor particle complex, and may be used as a patterned light-emitting layer in an electroluminescent apparatus or a photoluminescent apparatus, but is not limited thereto. Such electroluminescent apparatus or photoluminescent apparatus includes but is not limited to a QLED, an LED, a mini-LED, a micro-LED, a nano-LED, a QD-OLED, and the like.

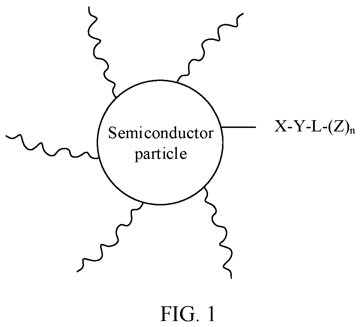

Refer to FIG. 1. The complex in embodiments of this application includes semiconductor particles and at least one new ligand compound, where the ligand compound is coordinated to a surface of the semiconductor particles. The ligand compound is optimized and selected, so that the complex has high photoactivity, and can reduce an exposure dose and shorten exposure duration, thereby improving patterning efficiency.

The ligand compound has a structure shown in the following formula (I):

X-Y-L-(Z)ₙ (I),

where
X is HOOC-, NH₂-, HOOOP-, HSS-, or HS-;
Y is selected from at least one of a single bond and an organic group, where the organic group is an aliphatic organic group, an aromatic organic group, and an organic group containing at least one of an ester group, an ether group, a carbonyl group, and an amide group;
L is an atom having a valence of at least three or an organic group having at least three chemical bonds;
Z is an organic group with an end-capping group being a crosslinkable group; and
n is an integer greater than or equal to 2.

It may be understood that "-" in formula (I) indicates that there is a linkage between any two adjacent elements of X, Y, L, and Z.

An end of a molecular main chain of the ligand compound contains a plurality of organic groups Z, and an end-capping group of each organic group Z is a crosslinkable group (for example, a photosensitive group). One end of the ligand compound is coordinated to the surface of the semiconductor particles through the X structure in formula (I), and the other end is linked to a plurality of organic groups Z with crosslinkable groups. The end of the ligand compound linked to the organic groups Z is defined as a free end. In a process of crosslinking and curing, free ends of ligand compounds linked to different semiconductor particle complexes are in contact with each other, to undergo crosslinking and curing through respective crosslinkable groups, to form a crosslinked structure.

In embodiments of this application, a plurality of organic groups Z (which may be referred to as branched chains Z herein) with crosslinking activity are linked to an end of a molecular main chain of the ligand compound, increasing a quantity of crosslinkable groups at the end of the molecular main chain of the ligand compound, which helps improve photoactivity of the ligand compound. In addition, the crosslinkable group is located at an end of each branched chain Z, increasing a chance of contact between crosslinkable groups on different semiconductor particle comlex, which helps contact between crosslinkable groups linked to different semiconductor particle complexes to undergo crosslinking and curing, further improving photoactivity, reducing an exposure dose, and shortening exposure duration, thereby improving patterning efficiency. Moreover, the increase of a quantity of crosslinkable groups can increase crosslinking density, which helps improve stability of a crosslinked network structure finally formed from the semiconductor particle complex.

Further, Z in formula (I) includes but is not limited to at least one of an alkenyl group, an alkynyl group, an allyl group, an acrylic group, an acryloyl group, an acrylamido group, an azido group, an azidophenyl group, a fluoro-substituted azidophenyl group, diazo carbon, benzophenone, and a cinnamyl group. For example, Z may be any one of the foregoing groups, or may be a series or parallel structure of the foregoing groups. End-capping groups of the foregoing organic groups are double bonds or triple bonds. These end-capping groups have a crosslinkable function. A plurality of the foregoing organic groups are contained in formula (I), increasing a quantity of crosslinkable groups, which can further improve photoactivity of the ligand compound.

Further, L in formula (I) is a pivotal structure for a plurality of branched chains Z to be linked to the molecular main chain of the ligand compound. According to different quantities of branched chains Z to be introduced, L may be an atom having a valence of at least three, or may be an organic group having at least three chemical bonds. Specifically, L may include carbon or nitrogen. The carbon atom is a tetravalent atom that can form four chemical bonds with another atom or organic group, and can be linked to two or three Zs with an end-capping group. For example, L may be 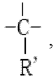 where R' may be H or another group; or L may be 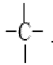 The nitrogen atom is a trivalent atom that can form three chemical bonds with another atom or organic group, and can be linked to two Zs with an end-capping group. For example, L may be 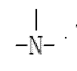 Therefore, when L in formula (I) includes carbon or nitrogen, photoactivity of the ligand compound can be improved, and the Z group has small steric hindrance for linkage, which helps introduce a plurality of Z groups in the ligand compound. When L is an organic group (such as an aliphatic organic group or an aromatic organic group) that can be linked to a plurality of branched chains, the plurality of linked branched chains Z may be linked to the same atom of the organic group (for example, when L is 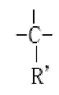 ), or may be linked to different atoms of the organic group.

Further, Y in formula (I) may be a saturated aliphatic organic group, and may specifically include but is not limited to at least one of a saturated aliphatic hydrocarbyl group, aliphatic ester group, aliphatic amide group, aliphatic oxycarbonyl group, and aliphatic ether group. By linking the saturated aliphatic organic group Y to the molecule of the ligand compound, flexibility of the molecular chain of the ligand compound can be improved, which helps a crosslinking reaction between terminal crosslinkable groups of different ligand compounds.

Further, when Y in formula (I) is a saturated aliphatic ester group with a number of carbon atoms of 1 to 22, the ligand compound has a structure shown in any one of chemical formulas (II) to (IV): 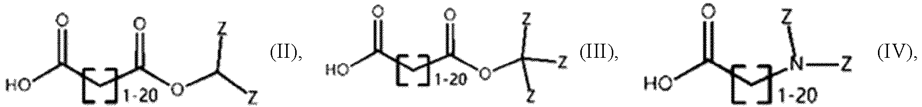 where
Z in formulas (II) to (IV) is the branched chain.

It can be seen from the structures of formulas (II) to (IV) that, in the foregoing three structures, two or three branched chains Z are linked to the free end of the ligand compound, and the branched chains Z are branched and distributed in a dendritic shape at the end of the molecular main chain of the ligand compound, increasing a quantity of crosslinkable active sites, further increasing a chance of contact between crosslinkable groups on different semiconductor particles, thereby improving photoactivity of the ligand compound, and also increasing crosslinking density, thereby improving stability of a final crosslinked structure formed from the semiconductor particle complex. In addition, as a quantity of branched chains with photo-crosslinking activity increases, a quantity of crosslinkable groups increases, and photoactivity and crosslinking efficiency of the ligand compound further increase. However, considering the impact of steric hindrance, the quantity of branched chains with photo-crosslinking activity cannot be increased without limitation. More branched chains may increase steric hindrance to a crosslinking reaction between each crosslinkable group and a crosslinkable group on another semiconductor particle complex. Therefore, the quantity of branched chains with photo-crosslinking activity needs to be controlled within a proper range. In an embodiment of this application, there are two or three branched chains. In addition, when Y in formula (I) is a saturated aliphatic ester group with a number of carbon atoms of 1 to 22, in a synthesis process, a raw material of the Y structure has only a carboxyl group at an end, and a location of an active linking site is determined and single, which helps introduction of the branched chain to the main chain, making a synthesis path easier to implement, so that the ligand compound with the structure shown in formula (I) designed in embodiments of this application is obtained.

Further, when the crosslinkable group in Z in formulas (II) to (IV) is a carbon-carbon double bond, the ligand compound has a structure shown in any one of chemical formulas (V) to (XVI):

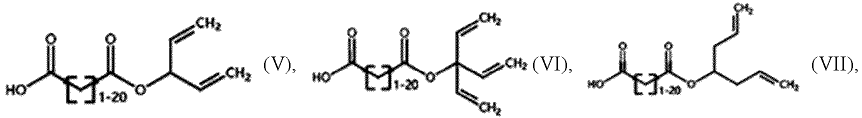

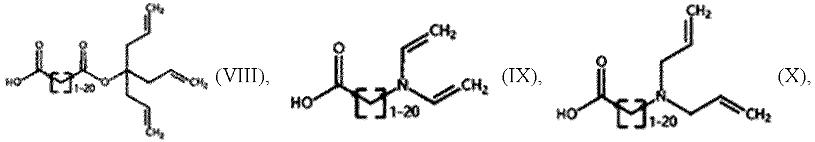

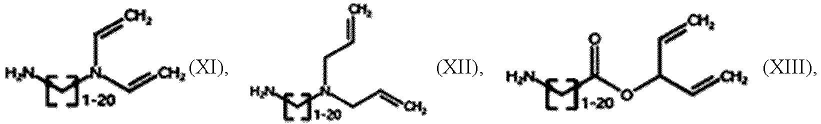

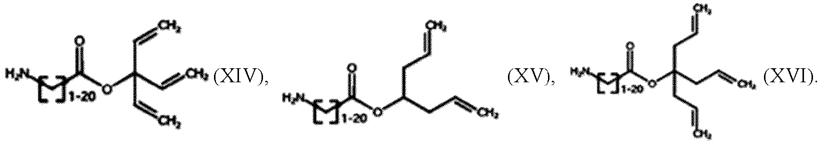

The semiconductor particles used in embodiments of this application are semiconductor nanocrystals (namely, quantum dots). The semiconductor nanocrystals are a new type of display materials, characterized by high luminous efficiency and narrow peak width, which can achieve low power consumption, highly collimated light emitting, a small size, and the like of an electroluminescent (photoluminescent) apparatus. The semiconductor nanocrystals may include all semiconductor nanoparticles prepared from a metal-containing precursor by a wet chemical method. For example, a corresponding metal-containing precursor may be added to an organic solvent with or without a dispersant. An available metal-containing precursor includes a metal elementary substance, a metal oxide, a metal salt, an organic metal compound, and the like. Nanoscale crystals are grown at a preset temperature to prepare semiconductor nanoparticles. Appropriate semiconductor nanoparticles used in embodiments of this application include nanocrystals of compounds of elements from groups II-IV, III-IV, and V, and mixtures thereof. Specifically, the semiconductor particles used in embodiments of this application include any one of CdS, CdSe, CdTe, ZnS, ZnSe, ZnTe, PbS, PbSe, PbTe, HgS, HgSe, HgTe, GaN, GaP, GaAs, InP, InAs, ZnO, SnO₂, TiO₂, In₂O₃, Ga₂O₃, SiO₂, NiO, MoO₃, WO₃, Cu₂O, CuO, Fe₃O₄, Au, Ag, carbon dots, CsPbCl₃, CsPbBr₃, CsPbI₃, CH₃NH₃PbCl₃, CH₃NH₃PbBr₃, and CH₃NH₃PbI₃, or a hybrid structure thereof. When the semiconductor particles are a hybrid structure, the hybrid structure may be a uniform hybrid structure, a gradient hybrid structure, a core-shell structure, or the like.

It may be understood that the semiconductor particles may be particles of another size, for example, micron-sized particles.

To reduce an amount of the foregoing new ligand compound and improve dispersion of the new ligand compound in an organic solvent, in embodiments of this application, the ligand compound shown in formula (I) is formulated with a conventional assistant ligand of existing semiconductor particles, that is, an assistant dispersing ligand (which may be a conventional ligand of semiconductor particles) is added to the complex, to obtain another complex of semiconductor nanoparticles. The assistant dispersing ligand is also coordinated to the surface of the semiconductor particles. There are a plurality of crosslinkable groups at the end of the molecular main chain of the new ligand compound synthesized in embodiments of this application. After a small amount of the ligand compound and the assistant dispersing ligand commonly used for the semiconductor particles are formulated, photoactivity of the formulated ligand can be significantly improved, an exposure dose can be reduced, and exposure duration can be shortened, thereby improving patterning efficiency. By formulating the ligand compound with the assistant dispersing ligand, the amount of the new ligand compound can be further reduced, thereby reducing costs. In addition, after a small amount of the new ligand compound is formulated with the assistant dispersing ligand, the small amount of the new ligand compound can be uniformly attached to the surface of the semiconductor particles, and dispersion of the semiconductor particle complex in an organic solvent can be improved, facilitating film formation.

Further, the assistant dispersing ligand may include but is not limited to at least one of ligands with a good dispersion function for nanoparticles, including fatty acid, unsaturated fatty acid, fatty amine, unsaturated fatty amine, alkylthiol, unsaturated alkylthiol, alkyl phosphonic acid, and unsaturated alkyl phosphonic acid, such as oleic acid, linoleic acid, and trioctylphosphine. By adding the assistant dispersing ligand of the foregoing type, dispersion of the new ligand compound synthesized in the foregoing embodiments can be improved, so that the new ligand compound can be uniformly linked to the surface of the semiconductor particles, further improving photoactivity of the semiconductor particles. In addition, by adding the assistant dispersing ligand, an amount of the new ligand compound can also be reduced while the photoactivity of the semiconductor particles is improved.

Further, a weight of the new ligand compound is 2% to 50% of a total weight of the assistant dispersing ligand and the new ligand compound, further 5% to 30%, and even further 10% to 20%. If an addition amount of the new ligand compound is excessively low (less than 2%), the improvement of the photoactivity of the semiconductor particles is limited. If an addition amount of the new ligand compound is excessively high (more than 50%), an amount of the assistant dispersing ligand is excessively low, which is not conducive to uniform dispersion of the new ligand compound, and the amount of the new ligand compound is increased, which increases costs of the new ligand compound.

The complex (namely, the semiconductor particle complex) in embodiments of this application is specifically prepared through the following steps:
Preparation is performed with a metal-containing precursor to obtain semiconductor particles (namely, semiconductor nanocrystals), the obtained semiconductor particles are then dispersed in an organic solvent to obtain a dispersion system, and the dispersion system is treated with the new ligand compound shown in formula (I). The dispersion system of the semiconductor particles is stirred or refluxed. Stirring or refluxing conditions (including time, temperature, a concentration of the foregoing formulated mixture, and the like) may be adjusted appropriately based on a type of the organic solvent for dispersion, the semiconductor particles, and the ligand compound coordinated to the surface of the semiconductor particles. In addition, process parameters for preparing the semiconductor particles may be controlled to control a type, size, and shape of the semiconductor particles.

Further, the dispersion system of the semiconductor particles is treated with the formulated mixture of the new ligand compound and the assistant dispersing ligand. Specifically, before the dispersion system of the semiconductor particles is treated, the semiconductor particles are first coordinated to a commonly used dispersing ligand (such as oleic acid or trioctylphosphine) on the surface. Then, the foregoing formulated mixture containing the new ligand compound and the commonly used dispersing ligand on the surface of the semiconductor particles undergo a ligand exchange reaction, to prepare the semiconductor particle complex in embodiments of this application.

An embodiment of this application further provides a semiconductor particle complex solution. The solution includes an organic solvent and the foregoing semiconductor particle complex dispersed in the organic solvent. A concentration of the semiconductor particle complex solution may be adjusted according to actual needs.

The complex can be dispersed in a conventional organic solvent, such as toluene or octane.

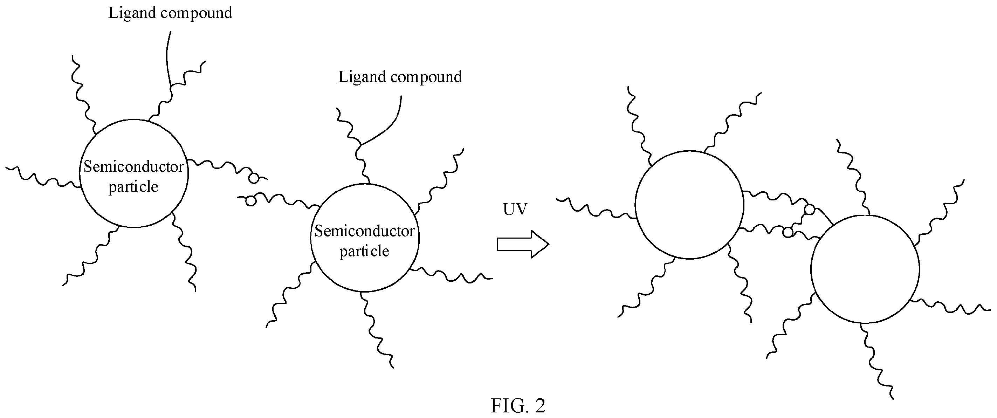

An embodiment of this application further provides a patterned light-emitting layer 100, which is specifically a patterned quantum dot film, but is not limited thereto. The patterned quantum dot film is obtained through photocuring and crosslinking of the foregoing complex. Different semiconductor particles in the formed patterned light-emitting layer 100 are linked together by a crosslinked structure, to form a crosslinked network structure, as shown in FIG. 2.

Because a plurality of branched chains Z with photo-crosslinking activity are linked to a free end of a molecular main chain of the new ligand compound in the complex, a quantity of crosslinkable groups on the surface of the complex is increased, thereby improving photoactivity of the ligand compound. In addition, the crosslinkable group is located at an end of each branched chain Z, increasing a chance of contact between crosslinkable groups on different semiconductor particles, which helps contact between crosslinkable groups linked to different complexes to undergo photo-crosslinking and curing, further improving photoactivity, reducing an exposure dose, and shortening exposure duration, thereby improving patterning efficiency. Moreover, due to the increase of a quantity of terminal crosslinkable groups, crosslinking density of a crosslinked network structure can be increased, improving stability of the crosslinked structure, and reducing damage to the crosslinked structure caused by a developing solution in a development process. The obtained patterned light-emitting layer has high pattern fineness, which helps improve resolution (up to an ultrahigh resolution of 3000 ppi or higher) of a display apparatus. In addition, a thickness of the patterned light-emitting layer is uniform and adjustable, and the patterned light-emitting layer can show good light-emitting features.

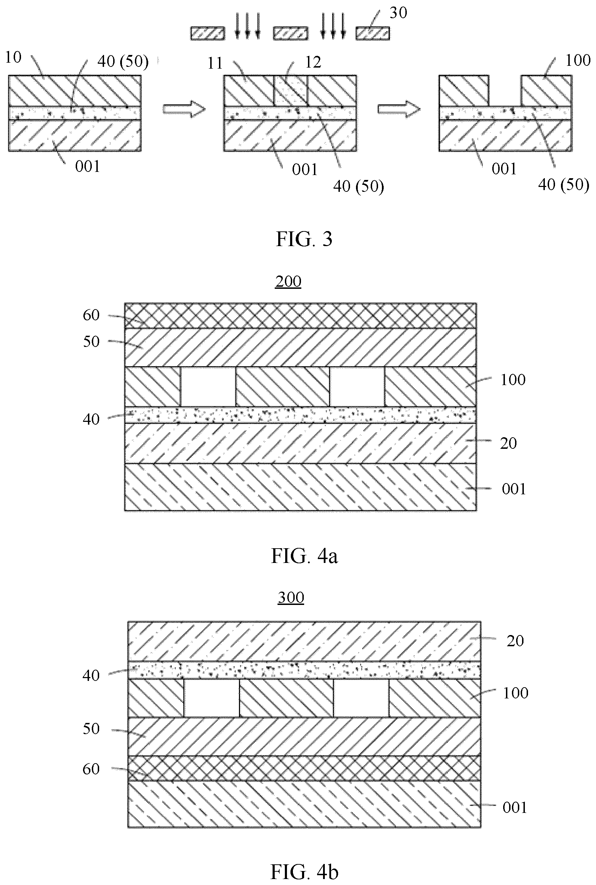

Refer to FIG. 3. An embodiment of this application further provides a method for preparing the patterned light-emitting layer 100, including: patterning a film 10 containing the foregoing complex, to obtain the patterned light-emitting layer 100, where the patterned light-emitting layer 100 includes a crosslinked structure obtained through crosslinking of the complex.

Further, the preparation method specifically includes the following steps:
Step S10: Provide the foregoing complex solution on a substrate 001, to form a complex film 10.

The foregoing complex is dissolved in an organic solvent, the substrate 001 is coated with the obtained complex solution to form a wet film, and then the organic solvent is removed under a heating condition to form the complex film 10.

In this step, the organic solvent is not specially limited, as long as the solvent can uniformly disperse the complex and can be easily removed after coating. Specifically, the organic solvent is selected from one of chloroform, chlorobenzene, toluene, tetrahydrofuran, dichloromethane, hexane, heptane, octane, nonane, decane, DMF, 4-hydroxy-4-methyl-2-pentanone, 2-ethoxyethanol, and 2-methoxyethanol, or a mixture thereof.

In this step, different from a general photoetching technique, curing can be performed using the semiconductor particle complex in embodiments of this application without a photoinitiator. It may be understood that, if necessary, a photoinitiator may be further added to assist in a crosslinking reaction. Specifically, an initiator that can form a free radical under light may be added, such as photoinitiators based on acetophenone, benzoin, benzophenone, phosphine oxide, and thiazolone, or some commercial photoinitiators.

In this step, a manner of coating for film formation may include but is not limited to spin coating, dip coating, spray coating, or blade coating.

In this step, a heating temperature for removing the organic solvent is 30°C to 300°C, to evaporate the organic solvent.

Before step S10, the preparation method further includes: coating the substrate 001 with a first functional layer solution or a second functional layer solution, and annealing at 30°C to 150°C in a nitrogen atmosphere for 30 min, to form a first functional layer 40 or a second functional layer 50 on the substrate. Subsequently, the first functional layer 40 or the second functional layer 50 is coated with the complex solution to form the complex film 10. The first functional layer 40 may be made of a polymer hole transport material, for example, poly[(9,9-dioctylfluorenyl-2,7-diyl)-co-(4,4'-(N-(4-sec-butylphenyl))diphenylamine)] (poly[(9,9-dioctylfluorenyl-2,7-diyl)-co-(4,4'-(N-(4-sec-butylphenyl))diphenylamine)], TFB). The second functional layer 50 may be made of an electron transport material of a metal oxide type, including but not limited to an oxide containing at least one of metal elements such as zinc, titanium, tin, magnesium, calcium, lithium, sodium, potassium, and indium.

Step S20: Expose the film 10 to obtain an exposed film.

The complex film 10 is selectively exposed to light by using a photoetching mask 30 with a desired pattern. In this case, a crosslinking reaction occurs in the ligand compound linked to the surface of the semiconductor particles in an exposure region 11. Through the crosslinking reaction, a network structure of the semiconductor nanoparticles can be formed. As a result, solubility of the complex in the exposure region 11 is different from that in a non-exposure region 12. Based on different solubilities, the exposed film 10 may be developed by using a developing agent to form a patterned light-emitting layer 100.

In this step, the exposure may be implemented through a contact or non-contact exposure process.

In this step, a light intensity (namely, an energy density of light) for exposing is 0.001 mW/cm² to 1000 mW/cm², further 10 mW/cm² to 800 mW/cm², even further 20 mW/cm² to 500 mW/cm², and even further 30 mW/cm² to 300 mW/cm², and time for exposing is 0.1 seconds to 600 seconds, further 1 second to 300 seconds, and even further 10 seconds to 180 seconds. Due to structural features of the ligand compound, a plurality of crosslinkable groups are introduced to the end of the molecular main chain, improving photoactivity of the ligand compound. Therefore, an exposure dose can be effectively reduced, and exposure duration can be shortened, thereby improving photo-crosslinking and curing efficiency. In addition, the exposure dose may be appropriately adjusted based on a thickness of the formed complex film 10.

In this step, a light source for exposing has an effective wavelength range of 200 nm to 500 nm, and further an effective wavelength range of 300 nm to 400 nm.

Step S30: Develop the exposed film to obtain the patterned light-emitting layer 100.

The exposed film is placed in a developing solution, so that a part of the film that does not undergo crosslinking and curing dissolves in the developing solution, forming the patterned light-emitting layer 100 with a specific pattern.

In this step, the used developing solution includes but is not limited to an organic solvent such as toluene, octane, or chloroform, or a weak acid solution, or a weak base solution, or pure water.

The patterned light-emitting layer is formed through a direct photoetching process. A process is simple, and a fine pattern can be formed without a photoresist. In addition, steps of the entire process are fewer than steps of a patterning process using a photoresist, with no need of a developing solution for the photoresist, which has little impact on the patterned light-emitting layer and a functional layer of a product, and is conducive to improving the quality and yield of the product.

Refer to FIG. 4a and FIG. 4b. An embodiment of this application further provides a light-emitting apparatus. The light-emitting apparatus includes the foregoing patterned light-emitting layer 100. Specifically, due to good light-emitting characteristics, the patterned light-emitting layer 100 provided in embodiments of this application may be used in an electroluminescent quantum dot apparatus or a photoluminescent quantum dot apparatus, such as a QLED, an LED, a mini-LED, a micro-LED, a nano-LED, or a QD-OLED, which is not limited thereto.

Specifically, the apparatus is an electroluminescent apparatus. According to different structures, the electroluminescent apparatus may be divided into an upright component 200 (FIG. 4a) and an inverted component 300 (FIG. 4b).

As shown in FIG. 4a, in the upright component 200, a base plate 001 is provided with an anode 20, and a first functional layer 40 may exist between the anode 20 and the patterned light-emitting layer 100. The first functional layer 40 may include one or more of a hole injection layer, a hole transport layer, and an electron blocking layer. A second functional layer 50 may exist between a cathode 60 and the patterned light-emitting layer 100. The second functional layer 50 may include one or more of an electron injection layer, an electron transport layer, and a hole blocking layer. For materials of the first functional layer 40 and the second functional layer 50, refer to the foregoing description.

As shown in FIG. 4b, in the inverted component 300, a base plate 001 is provided with a cathode 60, and a second functional layer 50 may exist between the cathode 60 and the patterned light-emitting layer 100. The second functional layer 50 may include one or more of an electron injection layer, an electron transport layer, and a hole blocking layer. A first functional layer 40 may exist between an anode 20 and the patterned light-emitting layer 100. The first functional layer 40 may include one or more of a hole injection layer, a hole transport layer, and an electron blocking layer. For materials of the first functional layer 40 and the second functional layer 50, refer to the foregoing description.

It may be understood that the base plate 001 in the foregoing two components 200 (300) may be provided with an active matrix or passive matrix drive circuit (not shown in the figure).

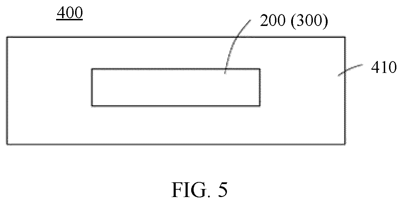

Refer to FIG. 5. An embodiment of this application further provides an electronic device 400. The electronic device 400 includes a housing 410 and the foregoing light-emitting apparatus in the housing 410. The light-emitting apparatus may be the foregoing upright component 200 or inverted component 300. Specifically, the electronic device 400 may be an augmented reality (augmented reality, AR) device, a virtual reality (virtual reality, VR) device, or the like.

The following further describes embodiments of this application by using specific embodiments.

### Synthesis Example 1

Synthesis of a ligand compound DE-C8 with a photosensitive function:
1.071 g of octanedioic acid is dispersed in 15 mL of anhydrous dichloromethane, and a dichloromethane solution containing 0.0631 g of 4-dimethylaminopyridine (DMAP) and a dichloromethane solution containing 1.415 g of 1-(3-dimethylaminopropyl)-3-ethylcarbodiimide hydrochloride (EDAC) are added in sequence at 0°C, where the whole processes are under nitrogen protection, and then stirred at 0°C for 1 h. Then, a dichloromethane solution containing 0.31 g of dienol is slowly added dropwise to the reaction solution and stirred at room temperature in a closed environment for 16 h to react, to obtain the ligand compound DE-C8.

The reaction is shown in the following formula:

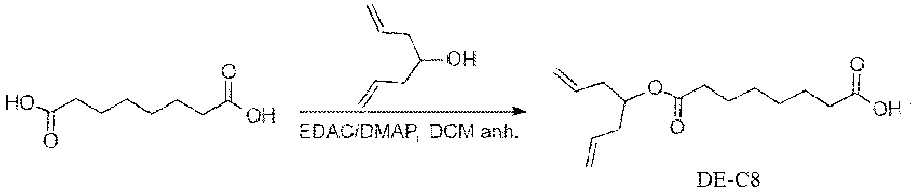

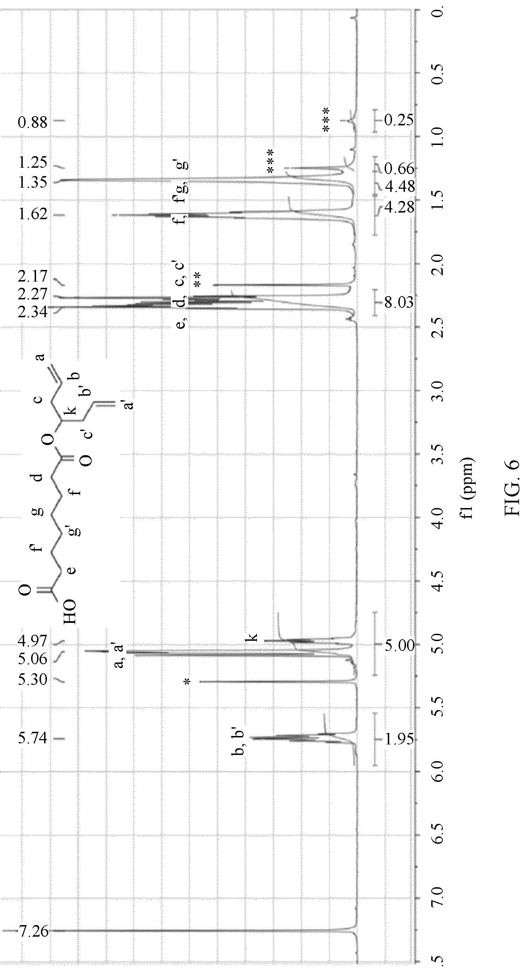

It can be learned from the nuclear magnetic resonance spectrum in FIG. 6 that the ligand compound DE-C8 is successfully synthesized.

### Synthesis Example 2

Synthesis of a ligand compound DE-C5 with a photosensitive function:
0.515 g of glutaric acid is dispersed in 15 mL of anhydrous dichloromethane, and a dichloromethane solution containing 0.04 g of DMAP and a dichloromethane solution containing 0.8962 g of EDAC are added in sequence at 0°C, where the whole processes are under nitrogen protection, and then stirred at 0°C for 1 h. Then, a dichloromethane solution containing 0.19 g of dienol is slowly added dropwise to the reaction solution and stirred at room temperature in a closed environment for 16 h to react, to obtain the ligand compound DE-C5.

The reaction is shown in the following formula:

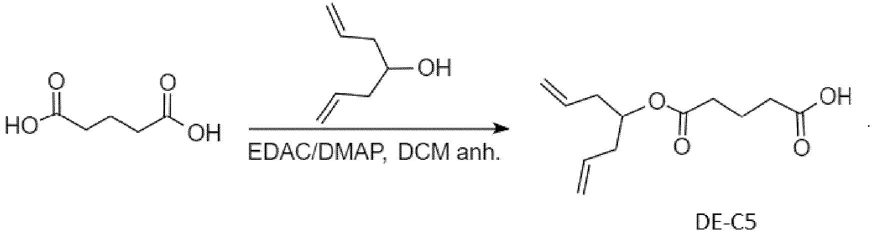

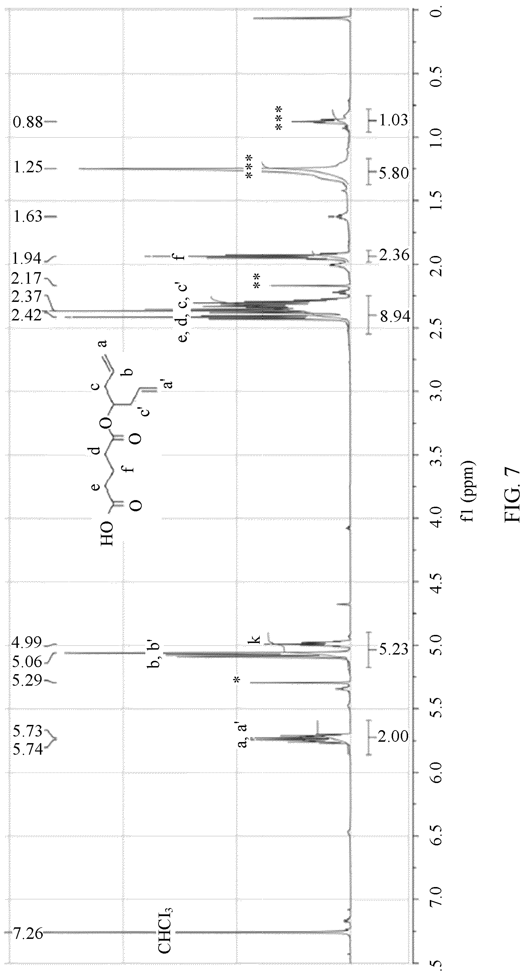

It can be learned from the nuclear magnetic resonance spectrum in FIG. 7 that the ligand compound DE-C5 is successfully synthesized.

### Synthesis Example 3

Synthesis of a ligand compound TE-C8 with a photosensitive function:
2.06 g of octanedioic acid is dispersed in 10 mL of anhydrous DCM, and a dichloromethane solution containing 0.1213 g of DMAP and a dichloromethane solution containing 2.72 g of EDAC are added in sequence at 0°C, where the whole processes are under nitrogen protection, and then stirred at 0°C for 1 h. Then, a dichloromethane (DCM) solution containing trienol (0.6 g) is slowly added dropwise to the reaction solution and stirred at room temperature in a closed environment for 17 h to react, to obtain the ligand compound TE-C8.

The reaction is shown in the following formula:

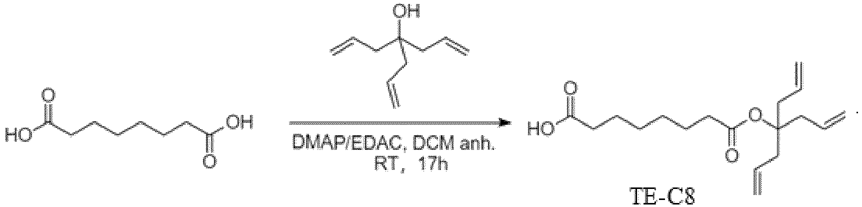

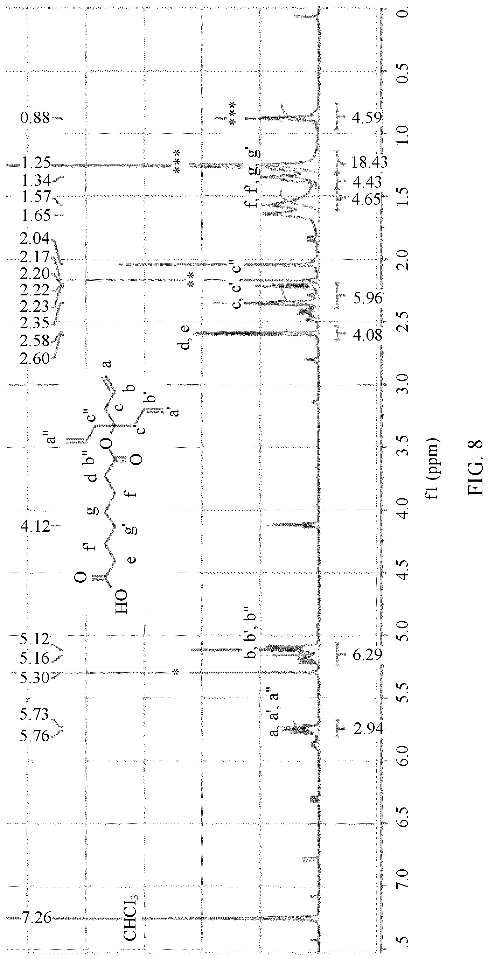

It can be learned from the nuclear magnetic resonance spectrum in FIG. 8 that the ligand compound TE-C8 is successfully synthesized.

### Synthesis Example 4

Synthesis of a ligand compound DE-A12 with a photosensitive function:
Step 1: 2 g of aminododecanoic acid and 1.86 mL of triethylamine are dissolved in 60 mL of methanol, and then 30 mL of di-tert-butyl dicarbonate (Boc) is added, and stirred at 60°C for 1 h, to obtain a Boc-protected product.

Step 2: The Boc-protected product is dissolved in anhydrous DCM, and a DCM solution containing 0.4986 g of dienol is added. 1 g of EDAC and 0.131 g of DMAP are added at 0°C, kept at 0°C for 1 h, and then kept at room temperature for 24 h.

Step 3: The reaction product obtained in step 2 is dissolved in 19 mL of ethanol, and 19 mL of triethylamine is added to react at room temperature for 12 h, to obtain the ligand compound DE-A12.

The reaction is shown in the following formula:

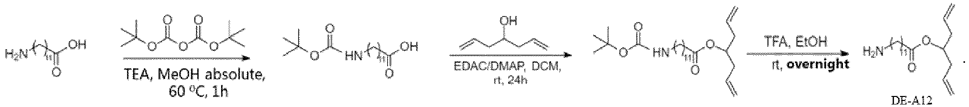

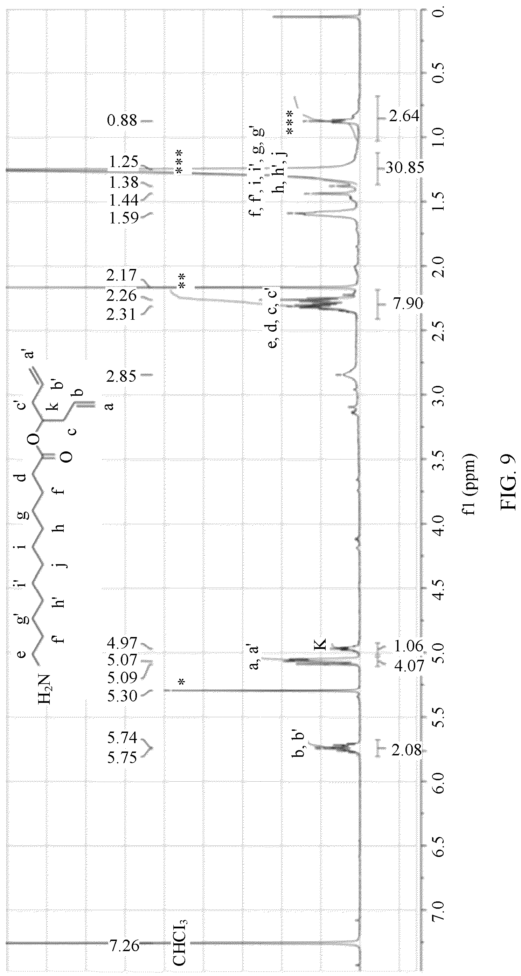

It can be learned from the nuclear magnetic resonance spectrum in FIG. 9 that the ligand compound DE-A12 is successfully synthesized.

### Preparation Example 1

Synthesis and preparation of semiconductor particles (namely, quantum dots) containing an oleic acid ligand on the surface:
Step 1: 5 mmol of Se powder and 5 mmol of S powder are dissolved in 5 mL of trioctylphosphine (trioctylphosphine, TOP) solution to obtain a (Se+S)-TOP precursor.

Step 2: 0.14 mmol of cadmium acetate, 3.41 mmol of zinc oxide, and 7 mL of oleic acid are added in a 50 mL three-necked flask, and heated under nitrogen protection to 150°C for 30 min to remove acetic acid and water. Next, 15 mL of ODE is added, and the reaction system is heated to 310°C. Then, 2 mL of (Se+S)-TOP precursor is injected, and the three-necked flask is cooled down to 300°C, to carry out a reaction for 15 min, to obtain CdZnSeS/ZnS nanoparticles.

Step 3: An excess mixed solution of methanol and acetone (3:1 vol) is added to precipitate the CdZnSeS/ZnS nanoparticles, and centrifuged at 8000 rpm for 5 min. The precipitate is dissolved in 5 mL of octane solution, precipitated again with 15 mL of acetone, and centrifuged at 8000 rpm for 5 min. This step is repeated once, to obtain semiconductor particle powder.

Step 4: The obtained semiconductor particle powder is prepared into a 50 mg/mL toluene solution for use.

### Preparation Example 2

Preparation of a semiconductor particle complex solution (a linolenic acid-semiconductor particle complex):
1 mL of the 50 mg/mL toluene solution of the semiconductor particles obtained in Preparation Example 1 is taken, and linolenic acid (50 mg) of the equal mass as the semiconductor particles is added and stirred at room temperature for 30 min, to exchange the oleic acid ligand on the semiconductor particles with linolenic acid, so that linolenic acid is coordinated to the surface of the semiconductor particles, to obtain a semiconductor particle complex. The semiconductor particle complex is precipitated once with excess ethanol to obtain a precipitate. The precipitate is redissolved in an octane solution and prepared into a 15 mg/mL semiconductor particle complex solution for spin coating, for use.

### Preparation Example 3

Step 1: 80 wt% of oleic acid and 20 wt% of DE-C8 in Synthesis Example 1 are mixed to form a mixed ligand. The used oleic acid is 40 mg and DE-C8 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the oleic acid and the ligand compound DE-C8 are coordinated to the surface of the semiconductor particles, to obtain a ligand-exchanged semiconductor particle complex (oleic acid + DE-C8). The exchanged semiconductor particle complex (oleic acid + DE-C8) is precipitated once with excess ethanol to obtain a precipitate. Then, the precipitate is prepared with octane into a 15 mg/mL solution for spin coating, for use.

### Preparation Example 4

Step 1: 80 wt% of linolenic acid and 20 wt% of DE-C8 in Synthesis Example 1 are mixed to form a mixed ligand. The used α-linolenic acid is 40 mg and DE-C8 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the linolenic acid and the ligand compound DE-C8 are coordinated to the surface of the semiconductor particles, to obtain a ligand-exchanged semiconductor particle complex (linolenic acid + DE-C8). The exchanged semiconductor particle complex (linolenic acid + DE-C8) is precipitated once with excess ethanol to obtain a precipitate. Then, the precipitate is prepared with octane into a 15 mg/mL solution for spin coating, for use.

### Preparation Example 5

Step 1: 80 wt% of oleic acid and 20 wt% of DE-C5 in Synthesis Example 2 are mixed to form a mixed ligand. The used oleic acid is 40 mg and DE-C5 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the oleic acid and the ligand compound DE-C5 are coordinated to the surface of the semiconductor particles, to obtain a ligand-exchanged semiconductor particle complex (oleic acid + DE-C5). The exchanged semiconductor particle complex (oleic acid + DE-C5) is precipitated once with excess ethanol to obtain a precipitate. Then, the precipitate is prepared with octane into a 15 mg/mL solution for spin coating, for use.

### Preparation Example 6

Step 1: 80 wt% of linolenic acid and 20 wt% of DE-C5 in Synthesis Example 2 are mixed to form a mixed ligand. The used α-linolenic acid is 40 mg and DE-C5 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the linolenic acid and the ligand compound DE-C5 are coordinated to the surface of the semiconductor particles, to obtain a ligand-exchanged semiconductor particle complex (linolenic acid + DE-C5). The exchanged semiconductor particle complex (linolenic acid + DE-C5) is precipitated once with excess ethanol to obtain a precipitate. Then, the precipitate is prepared with octane into a 15 mg/mL solution for spin coating, for use.

### Preparation Example 7

Step 1: 80 wt% of oleic acid and 20 wt% of TE-C8 in Synthesis Example 3 are mixed to form a mixed ligand. The used oleic acid is 40 mg and TE-C8 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the oleic acid and the ligand compound TE-C8 are coordinated to the surface of the semiconductor particles, to obtain a ligand-exchanged semiconductor particle complex (oleic acid + TE-C8). The exchanged semiconductor particle complex (oleic acid + TE-C8) is precipitated once with excess ethanol to obtain a precipitate. Then, the precipitate is prepared with octane into a 15 mg/mL solution for spin coating, for use.

### Preparation Example 8

Step 1: 80 wt% of linolenic acid and 20 wt% of TE-C8 in Synthesis Example 3 are mixed to form a mixed ligand. The used α-linolenic acid is 40 mg and TE-C8 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the linolenic acid and the ligand compound TE-C8 are coordinated to the surface of the semiconductor particles, to obtain a ligand-exchanged semiconductor particle complex (linolenic acid + TE-C8). The exchanged semiconductor particle complex (linolenic acid + TE-C8) is precipitated once with excess ethanol to obtain a precipitate. Then, the precipitate is prepared with octane into a 15 mg/mL solution for spin coating, for use.

### Preparation Example 9

Step 1: 80 wt% of oleic acid and 20 wt% of DE-A12 in Synthesis Example 4 are mixed to form a mixed ligand. The used oleic acid is 40 mg and DE-A12 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the oleic acid and the ligand compound DE-A12 are coordinated to the surface of the semiconductor particles, to obtain a ligand-exchanged semiconductor particle complex (oleic acid + DE-A12). The exchanged semiconductor particle complex (oleic acid + DE-A12) is precipitated once with excess ethanol to obtain a precipitate. Then, the precipitate is prepared with octane into a 15 mg/mL solution for spin coating, for use.

### Preparation Example 10

Step 1: 80 wt% of linolenic acid and 20 wt% of DE-A12 in Synthesis Example 4 are mixed to form a mixed ligand. The used α-linolenic acid is 40 mg and DE-A12 is 10 mg.

Step 2: 1 mL of the 50 mg/mL toluene solution of the semiconductor particles obtained in Preparation Example 1 is taken, and the mixed ligand obtained in step 1 is added and stirred at room temperature for 30 min, so that the linolenic acid and the ligand compound DE-A12 are coordinated to the surface of the semiconductor particles, to obtain a ligand-exchanged semiconductor particle complex (linolenic acid + DE-A12). The exchanged semiconductor particle complex (linolenic acid + DE-A12) is precipitated once with excess ethanol to obtain a precipitate. Then, the precipitate is prepared with octane into a 15 mg/mL solution for spin coating, for use.

### Embodiment 1

Step 1: A clean substrate (for example, a glass sheet) is spin-coated with a 6 mg/mL TFB in chlorobenzene solution at a rotational speed of 2800 rpm and an acceleration of 900 rpm for 30s, and then annealed at 150°C in a nitrogen atmosphere for 30 min, to obtain a first functional layer.

Step 2: The first functional layer is spin-coated with the semiconductor particle complex (oleic acid + DE-C8) solution obtained in Preparation Example 3, at a rotational speed of 2500 rpm and an acceleration of 800 rpm for 30s, and a solvent is removed, to obtain a nanoparticle complex film.

Step 3: Exposure: The nanoparticle complex film is irradiated with a 365 nm LED flat panel light source (with an energy density of about 30 mW/cm²) to undergo crosslinking for 1 min to 5 min.

Step 4: Development: The exposed semiconductor particle complex film and the glass sheet are immersed in an octane solution, and left to stand for 1 min, to remove an uncrosslinked region in the semiconductor particle complex film, to finally obtain a patterned quantum dot film.

### Embodiment 2

A difference from Embodiment 1 lies in that: a semiconductor particle complex solution used is the semiconductor particle complex (linolenic acid + DE-C8) solution obtained in Preparation Example 4, to prepare a patterned quantum dot film.

### Embodiment 3

A difference from Embodiment 1 lies in that: a semiconductor particle complex solution used is the semiconductor particle complex (oleic acid + DE-C5) solution obtained in Preparation Example 5, to prepare a patterned quantum dot film.

Absorbance on a UV-VIS absorption curve of the obtained patterned light-emitting layer is not significantly lower than that of the light-emitting layer before exposure and development.

### Embodiment 4

A difference from Embodiment 1 lies in that: a semiconductor particle complex solution used is the semiconductor particle complex (linolenic acid + DE-C5) solution obtained in Preparation Example 6, to prepare a patterned quantum dot film.

Absorbance on a UV-VIS absorption curve of the obtained patterned light-emitting layer is not significantly lower than that of the light-emitting layer before exposure and development.

### Embodiment 5

A difference from Embodiment 1 lies in that: a semiconductor particle complex solution used is the semiconductor particle complex (oleic acid + TE-C8) solution obtained in Preparation Example 7, to prepare a patterned quantum dot film.

Absorbance on a UV-VIS absorption curve of the obtained patterned light-emitting layer is not significantly lower than that of the light-emitting layer before exposure and development.

### Embodiment 6

A difference from Embodiment 1 lies in that: a semiconductor particle complex solution used is the semiconductor particle complex (linolenic acid + TE-C8) solution obtained in Preparation Example 8, to prepare a patterned quantum dot film.

Absorbance on a UV-VIS absorption curve of the obtained patterned light-emitting layer is not significantly lower than that of the light-emitting layer before exposure and development.

### Embodiment 7

A difference from Embodiment 1 lies in that: a semiconductor particle complex solution used is the semiconductor particle complex (oleic acid + DE-A12) solution obtained in Preparation Example 9, to prepare a patterned quantum dot film.

Absorbance on a UV-VIS absorption curve of the obtained patterned light-emitting layer is not significantly lower than that of the light-emitting layer before exposure and development.

### Embodiment 8

A difference from Embodiment 1 lies in that: a semiconductor particle complex solution used is the semiconductor particle complex (linolenic acid + DE-A12) solution obtained in Preparation Example 10, to prepare a patterned quantum dot film.

Absorbance on a UV-VIS absorption curve of the obtained patterned light-emitting layer is not significantly lower than that of the light-emitting layer before exposure and development.

### Comparative Example 1

A difference from Embodiment 1 lies in that: the semiconductor particle complex solution containing only the oleic acid ligand obtained in Preparation Example 1 is used to prepare a patterned quantum dot film, and crosslinking time in step 3 is 1 min to 10 min.

### Comparative Example 2

A difference from Embodiment 2 lies in that: the semiconductor particle complex solution containing only the α-linolenic acid ligand obtained in Preparation Example 2 is used to prepare a patterned quantum dot film, and crosslinking time in step 3 is 1 min to 10 min.

The following describes crosslinking effect of a semiconductor particle complex by measuring an ultraviolet-visible light (UV-VIS) absorption curve of a patterned light-emitting layer. Compared with a semiconductor particle complex film without exposure-development treatment, if the absorbance on the UV-VIS absorption curve of the patterned light-emitting layer obtained after exposure-development is significantly reduced, the semiconductor particle complex has poor photo-crosslinking effect, and consequently the semiconductor particle complex is ished off by the developing solvent. On the contrary, if the absorbance on the UV-VIS absorption curve of the obtained patterned light-emitting layer is not significantly reduced, photo-crosslinking effect is good.

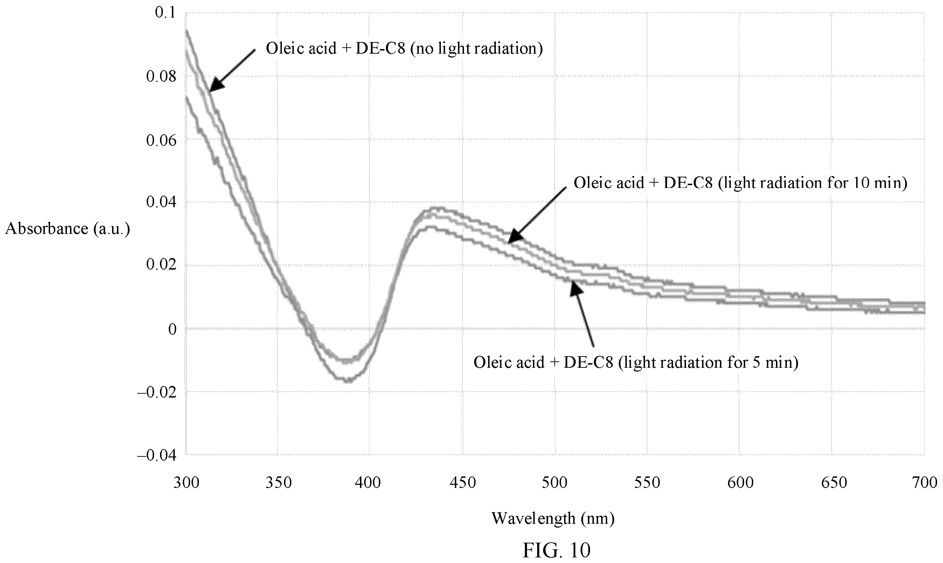

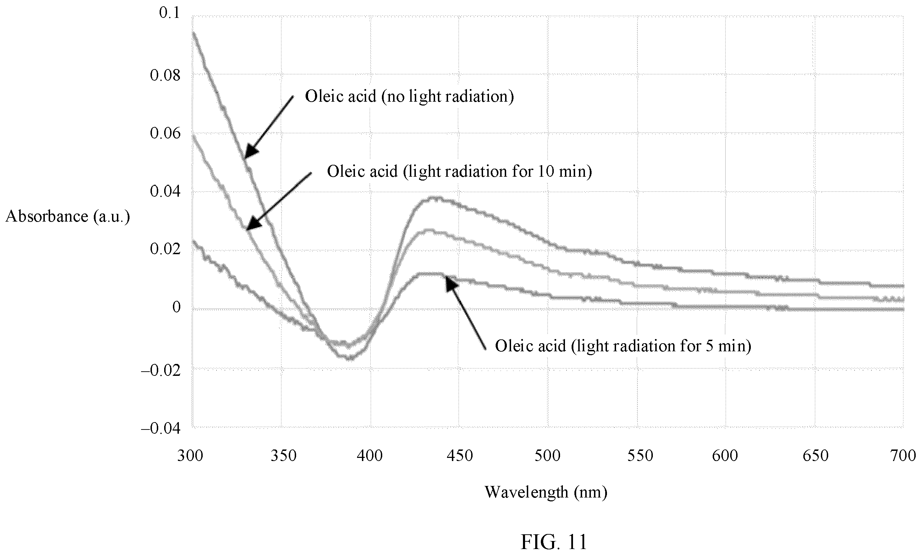

FIG. 10 and FIG. 11 show ultraviolet-visible light absorption curves of the patterned light-emitting layers obtained in Embodiment 1 and Comparative Example 1. It can be learned through comparison between FIG. 10 and FIG. 11 that, under the same light and elution conditions, the mixed ligand of oleic acid + 20 wt% DE-C8 is used as a quantum dot ligand in Embodiment 1 of this application, and compared with Comparative Example 1 in which only oleic acid is used as the ligand, the mixed ligand in this embodiment of this application has stronger photo-crosslinking effect and higher absorbance remaining after ishing. The reasons are as follows. The oleic acid ligand has low photo-crosslinking activity, so that an amount of the oleic acid ligand left on the quantum dots after ishing is small. However, two terminal double bonds of the ligand compound DE-C8 synthesized in the synthesis example of this application are exposed on the surface of the quantum dots, and can be more easily crosslinked to double bonds on a surface of other quantum dots under light. Therefore, by adding a small amount of the ligand compound DE-C8, photo-crosslinking activity of the mixed ligand can be effectively improved, an amount of the ligand compound left on the quantum dots after ishing is large, and photo-crosslinking effect is better.

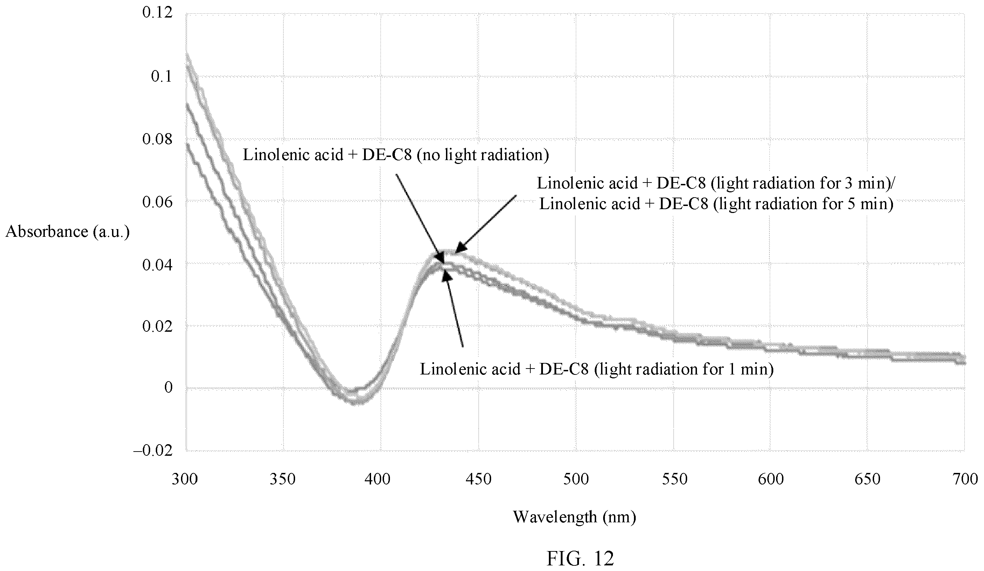

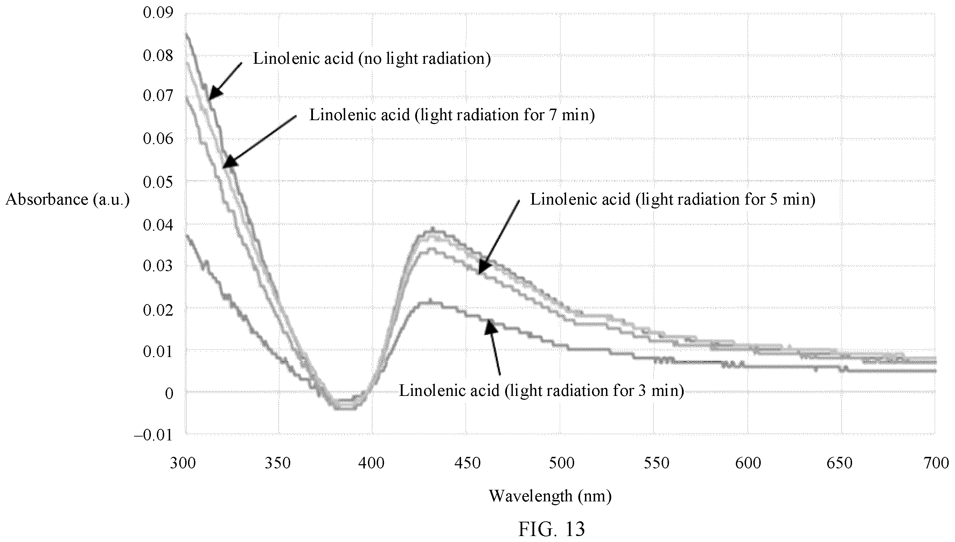

FIG. 12 and FIG. 13 show ultraviolet-visible light absorption curves of the patterned light-emitting layers obtained in Embodiment 2 and Comparative Example 2. It can be learned through comparison between FIG. 12 and FIG. 13 that, under the same light and elution conditions, the mixed ligand of α-linolenic acid + 20 wt% DE-C8 is used as a quantum dot ligand in Embodiment 2 of this application, and compared with Comparative Example 2 in which only α-linolenic acid is used as the ligand, the mixed ligand in this application has stronger photo-crosslinking effect and higher absorbance remaining after ishing. The reasons are as follows. The linolenic acid ligand has low photo-crosslinking activity, so that an amount of the linolenic acid ligand left on the quantum dots after ishing is small. However, two terminal double bonds of the ligand compound DE-C8 synthesized in the synthesis example of this application are exposed on the surface of the quantum dots, and can be more easily crosslinked to double bonds on a surface of other quantum dots under light. Therefore, by adding a small amount of the ligand compound DE-C8, photo-crosslinking activity of the mixed ligand can be effectively improved, an amount of the ligand compound left on the quantum dots after ishing is large, and photo-crosslinking effect is better.

In addition, as shown in FIG. 10, in Embodiment 1, the mixed ligand of oleic acid + 20 wt% DE-C8 is used as the quantum dot ligand, and as the crosslinking time increases, the absorbance increases, indicating that a specific increase in the crosslinking time can increase a crosslinking degree of the ligand. As shown in FIG. 12, in Embodiment 2, the mixed ligand of α-linolenic acid + 20 wt% DE-C8 is used as the quantum dot ligand, and within a fixed time range, as the crosslinking time increases, the absorbance increases, but an absorbance curve obtained through crosslinking for 3 minutes and an absorbance curve obtained through crosslinking for 5 minutes almost overlap. It can be learned that the use of the mixed ligand of α-linolenic acid + 20 wt% DE-C8 as the quantum dot ligand can significantly shorten exposure time and improve crosslinking efficiency.

Compared with Comparative Example 1 and Comparative Example 2, in each of Embodiment 3 to Embodiment 8, oleic acid and the ligand compound synthesized in the synthesis example of this application are mixed to be used as a quantum dot ligand, and compared with using only oleic acid or α-linolenic acid as the ligand, the mixed ligand with the ligand compound synthesized in the synthesis example of this application added has stronger photo-crosslinking effect and higher absorbance remaining after ishing. The reasons are as follows. The oleic acid ligand has low photo-crosslinking activity, so that an amount of the oleic acid ligand left on the quantum dots after ishing is small. However, two or three terminal double bonds of each of the ligand compounds in Synthesis Example 2 to Synthesis Example 4 of this application are exposed on the surface of the quantum dots, and can be more easily crosslinked to double bonds on a surface of other quantum dots under light. Therefore, by adding a small amount of each of the ligand compounds in Synthesis Example 2 to Synthesis Example 4, photo-crosslinking activity of the mixed ligand can be effectively improved, an amount of the ligand compound left on the quantum dots after ishing is large, and photo-crosslinking effect is better.

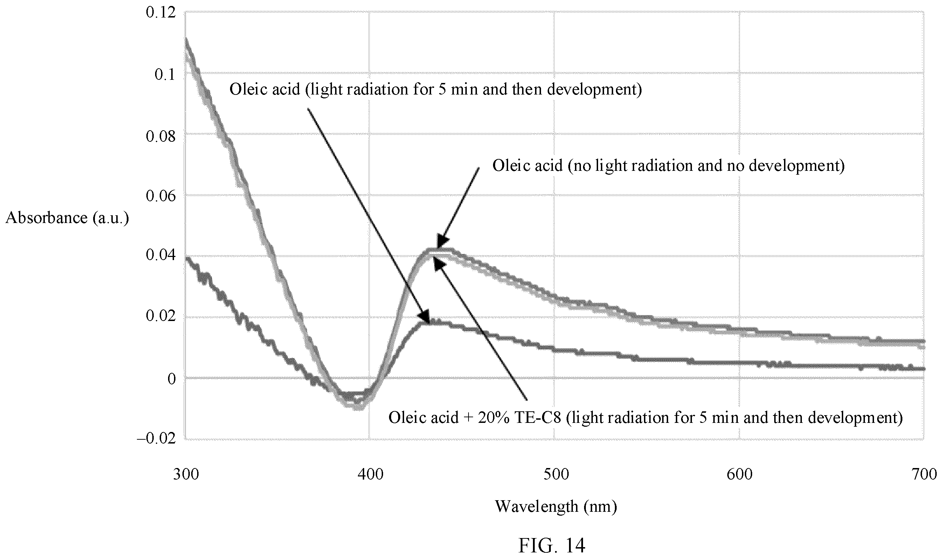

In addition, through comparison between Embodiment 1 and Embodiment 5, it can be learned with reference to FIG. 10 and FIG. 14 that, as a quantity of branched chains with photo-crosslinking activity increases (where there are two Zs in Embodiment 1, and there are three Zs in Embodiment 5), a quantity of crosslinkable groups increases, and photoactivity and crosslinking efficiency of the ligand compound further increase. For example, through comparison between the spectrum shown in FIG. 10 and the spectrum shown in FIG. 14, after 5 min of photo-crosslinking, a crosslinking degree of TE-C8 is higher than a crosslinking degree of DE-C8.

It needs to be noted that the foregoing descriptions are merely specific implementations of this application, but are not intended to limit the protection scope of this application. Any variation or replacement readily figured out by a person skilled in the art within the technical scope disclosed in this application shall fall within the protection scope of this application. In the case of no conflict, the implementations of this application and the features in the implementations may be mutually combined. Therefore, the protection scope of this application shall be subject to the protection scope of the claims.

## Claims

1. A ligand compound, wherein the ligand compound has a structure shown in the following formula (I):
X-Y-L-(Z)ₙ (I),
wherein
X is HOOC-, NH₂-, HOOOP-, HSS-, or HS-;
Y is at least one of a single bond, an aliphatic organic group, an aromatic organic group, and an organic group containing at least one of an ester group, an ether group, a carbonyl group, and an amide group;
L is an atom having a valence of at least three or an organic group having at least three chemical bonds;
Z is an organic group with an end-capping group being a crosslinkable group; and
n is an integer greater than or equal to 2.

2. The ligand compound according to claim 1, wherein Z in formula (I) is selected from at least one of an alkenyl group, an alkynyl group, an allyl group, an acrylic group, an acryloyl group, an acrylamido group, an azido group, an azidophenyl group, a fluoro-substituted azidophenyl group, diazo carbon, benzophenone, and a cinnamyl group.

3. The ligand compound according to claim 1, wherein L in formula (I) comprises carbon or nitrogen.

4. The ligand compound according to claim 1, wherein Y in formula (I) is a saturated aliphatic organic group, and is selected from at least one of a saturated aliphatic hydrocarbyl group, aliphatic ester group, aliphatic amide group, aliphatic oxycarbonyl group, and aliphatic ether group.

5. The ligand compound according to claim 4, wherein the ligand compound has a structure shown in any one of chemical formulas (II) to (IV): 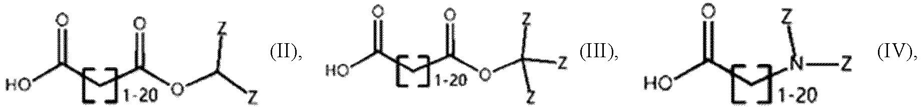 wherein
Z in formulas (II) to (IV) is an organic group with an end-capping group being a crosslinkable group.

6. The ligand compound according to claim 5, wherein the crosslinkable group is a carbon-carbon double bond, and the ligand compound has a structure shown in any one of chemical formulas (V) to (XVI):

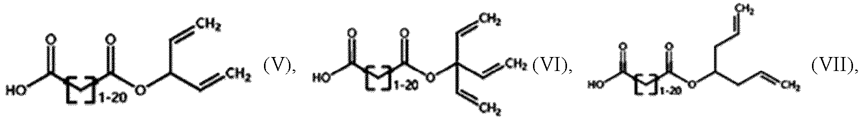

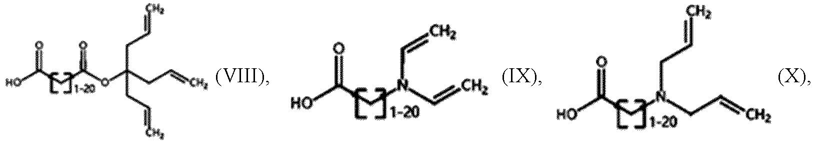

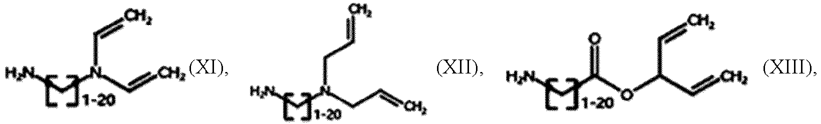

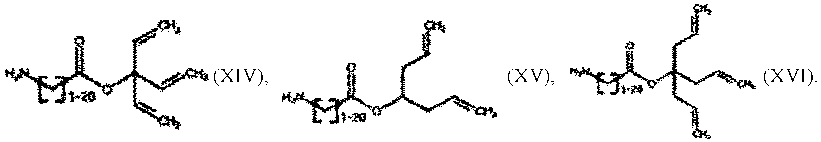

7. A complex, comprising semiconductor particles and at least one of the ligand compounds according to any one of claims 1 to 6, wherein the ligand compound is coordinated to a surface of the semiconductor particles.

8. The complex according to claim 7, wherein the semiconductor particles comprise at least one of CdS, CdSe, CdTe, ZnS, ZnSe, ZnTe, PbS, PbSe, PbTe, HgS, HgSe, HgTe, GaN, GaP, GaAs, InP, InAs, ZnO, SnO₂, TiO₂, In₂O₃, Ga₂O₃, SiO₂, NiO, MoO₃, WO₃, Cu₂O, CuO, Fe₃O₄, Au, Ag, carbon dots, CsPbCl₃, CsPbBr₃, CsPbI₃, CH₃NH₃PbCl₃, CH₃NH₃PbBr₃, and CH₃NH₃PbI₃.

9. The complex according to claim 7, further comprising an assistant dispersing ligand, wherein the assistant dispersing ligand is coordinated to the surface of the semiconductor particles, and a weight of the ligand compound is 2% to 50% of a total weight of the assistant dispersing ligand and the ligand compound.

10. The complex according to claim 9, wherein the weight of the ligand compound is 5% to 30% of the total weight of the assistant dispersing ligand and the ligand compound.

11. The complex according to claim 9, wherein the assistant dispersing ligand comprises at least one of fatty acid, unsaturated fatty acid, fatty amine, unsaturated fatty amine, alkylthiol, unsaturated alkylthiol, alkyl phosphonic acid, and unsaturated alkyl phosphonic acid.

12. A complex solution, comprising an organic solvent and the complex according to any one of claims 7 to 11 dispersed in the organic solvent.

13. A patterned light-emitting layer, comprising a crosslinked structure, wherein the crosslinked structure is obtained through crosslinking of the complex according to any one of claims 7 to 11.

14. A method for preparing a patterned light-emitting layer, comprising the following step:
patterning a film containing the complex according to any one of claims 7 to 11, to obtain the patterned light-emitting layer, wherein the patterned light-emitting layer comprises a crosslinked structure obtained through crosslinking of the complex.

15. The method for preparing the patterned light-emitting layer according to claim 14, comprising the following steps: exposing the film containing the complex according to any one of claims 7 to 11, to obtain an exposed film; and
developing the exposed film to obtain the patterned light-emitting layer.

16. The preparation method according to claim 15, wherein time for exposing is 0.1 seconds to 600 seconds.

17. The preparation method according to claim 15, wherein a light intensity for exposing is 0.001 mW/cm² to 1000 mW/cm².

18. A light-emitting apparatus, comprising the patterned light-emitting layer according to claim 13 or the patterned light-emitting layer prepared by the method for preparing the patterned light-emitting layer according to any one of claims 14 to 17.

19. An electronic device, comprising a housing and the light-emitting apparatus according to claim 18 on the housing.
